# FASCICULE DE BREVET EUROPEEN

(11) **EP 0 783 319 B1**
(45) Date de publication et mention de la délivrance du brevet: **14.05.2003**
(21) Numéro de dépôt: 95932794.1
(22) Date de dépôt: 26.09.1995
(51) Int. Cl.: A61K 38/14, A61P 37/00

(54) **COMPOSITIONS DE MURAMYLPEPTIDES INHIBANT LA REPLICATION DU VIH**
MURAMYLPEPTIDE ZUSAMMENSETZUNGEN FÜR INHIBIERUNG VON HIV REPLIKATION
MURAMYL PEPTIDE COMPOSITIONS FOR INHIBITING HIV REPLICATION

(30) Priorité: 26.09.1994 FR 9411460
(43) Date de publication de la demande: 16.07.1997
(73) Titulaire: VACSYN S.A., 75015 Paris (FR)
(72) Inventeur: BAHR, Georges, F-92800 Puteaux (FR)
(74) Mandataire: Gutmann, Ernest
(86) Numéro de dépôt international: FR9501239
(87) Numéro de publication internationale: WO96009837

(56) Documents cités:
- FR-A- 2 692 148
- JOURNAL OF MEDICINAL CHEMISTRY, vol. 25, no. 1, WASHINGTON US, pages 87-90, LEFRANCIER P. ET AL 'Apyrogenic, Adjuvant-Active N-Acetylmuramyl-dipeptides'
- INT. J. IMMUNOTHER. (1993), 9(3), 143-50 , 1993 MASIHI, K. N. ET AL 'Effect of the synthetic immunomodulator adamantylamide dipeptide on replication of human immunodeficiency virus alone and in combination with azidothymidine'
- AIDS RES. HUM. RETROVIRUSES (1990), 6(10), 1157-61, 1990 LAZDINS, JANIS K. ET AL 'The lipophilic muramyl peptide MTP-PE is a potent inhibitor of HIV replication in macrophages' cité dans la demande
- CLINICAL AND EXPERIMENTAL IMMUNOLOGY, vol. 90, 1992 pages 188-193, SCHRECK R. ET AL. 'Selection of a muramyl peptide based on its lack of activation of nuclear factor-kappaB as a potential adjuvant for AIDS vaccines'
- AIDS RES. HUM. RETROVIRUSES (1990), 6(3), 393-9 , 1990 MASIHI, K. NOEL ET AL 'Muramyl dipeptide inhibits replication of human immunodeficiency virus in vitro' cité dans la demande

## Description

Le syndrome d'immunodéficience acquise (SIDA) est une maladie dévastatrice causée par une infection par le rétrovirus VIH. Beaucoup d'efforts ont été consacrés à trouver des médicaments capables d'inhiber la réplication du virus. Mais peu de succès significatifs ont été obtenus jusqu'à ce jour. Bien que le VIH puisse infecter beaucoup de cellules différentes, la maladie est majoritairement causée par la destruction et/ou le dysfonctionnement d'une sous-population de lymphocytes appelés cellules T auxiliaires. On attribue depuis peu la persistance de l'infection par le virus à sa capacité à infecter une autre population importante de cellules, la lignée monocyte/macrophage, qui servirait de réservoir pour un relargage continu du virus. Le rôle important joué par cette lignée par VIH dans la persistance et la progression de la maladie a été expliquée par 1) l'isolement de variants monocytotropes du VIH des leucocytes du sang circulant et des macrophages tissulaires des sujets infectés à tous les stades de l'infection (J. Virology, ; Vol. 65, pages 356-363, 1991) et 2) la corrélation directe entre une absence de dysfonctionnement de l'immunité systémique chez l'hôte infecté et une absence de réplication virale dans la lignée monocyte/macrophage (J. infectious diseases, Vol. 168, pages 1140-1147, 1993). De plus, l'inhibition d'une infection produisant du virus dans les monocytes semble être liée dans une grande mesure à l'inhibition de la prolifération monocytaire, suggérant que la réplication du virus dépend d'une étape préalable obligatoire de prolifération de la cellule monocytaire haute. Ainsi, la prolifération de cette population serait-elle un passage obligé pour la manifestation du caractère infectieux du VIH. Ainsi l'hypothèse a-t-elle été formulée que des substances capables d'inhiber la réplication monocytaire pourraient aussi inhiber la réplication de VIH (J. Clinical Investigation, Vol. 89, pages 1154-1160, 1992).

Les muramylpeptides sont des copies synthétiques de la paroi bactérienne et ont été trouvés capables de très nombreuses activités immunopharmacologiques sur la lignée monocyte/macrophage (Federation proceedings, Vol. 45, pages 2541-2544, 1986). De plus, la molécule initiale, la N-acétyl-muramyl-L-alanyl-D- Isoglutamine (Nac-Mur-L-Ala-DisoGln) encore appelée Muramyl dipeptide ou MDP, a été décrite capable d'inhiber la prolifération des macrophages de cobayes (Cellular Immunology, Vol. 89, pages 427-438, 1984). Dans une autre étude utilisant des lignées cellulaires établies de lymphocytes ou de cellules de type monocytaires, le MDP a été trouvé doué de la capacité d'inhiber partiellement la réplication de HIV, lorsqu'il est utilisé in vitro à des doses très élevées 1000 µg/ml (AIDS Research and Human Retroviruses, Vol. 6, pages 393/394, 1990). Cependant outre que l'utilisation du MDP en clinique humaine est difficilement envisageable à cause des effets secondaires qu'il induit, les effets observés, même à ces doses élevées dans le système expérimental utilisé, ne préjugeraient d'aucune efficacité thérapeutique vis-à-vis de l'infection par le VIH. Lazdins et al (AIDS Research and Human Retroviruses, Vol. 6, pages 1157-1161, 1990),ont montré in vitro des propriétés similaires d'inhibition de la réplication du VIH pour un muramylpeptide ayant un meilleur index thérapeutique que le MDP : le MTP-PE. Cette molécule sous forme libre a été ajoutée de façon répétitive avant et après l'infection par VIH à des cultures de macrophages issus de monocytes humains mis en culture. Mais elle n'a pu, dans ces conditions, induire qu'une réduction partielle de la réplication virale. Il faut souligner que, le MTP-PE n'a été capable, ni à l'état libre, ni incorporé dans des liposomes, de provoquer une suppression totale de la réplication virale. En outre son activité ne peut s'exercer que si ce composé est présent le jour de l'infection de la culture cellulaire par le virus. Si le composé est ajouté un jour avant ou 4 jours après la culture, son activité est minimum.

Shcreck et al, *Clin. Exp. Immunol.* 90 pp. 188-193 (1992) décrit la sélection d'un muramyl peptide en tant qu'adjuvant potentiel pour des vaccins contre le SIDA, la sélection étant basée sur l'absence d'activation de facteur -κB nucléaire. Seul le MDP (thr)-GDP a été sélectionné.

Ces résultats ne rendent que plus étonnants ceux qui ont été obtenus avec une autre catégorie de muramylpeptides, qui se sont avérés permettre une inhibition complète de la prolifération de VIH, notamment dans des cultures primaires de monocytes, et ce à des doses beaucoup plus faibles. Leur toxité moindre s'ajoutant à ces effets favorables, les rend donc aptes à la constitution de médicaments aptes à prévenir ou traiter des SIDA et/ou des syndromes qui s'y rapportent.

L'invention est plus particulièrement relative à l'utilisation pour la constitution de médicaments inhibant la réplication de rétrovirus de l'immunodéficience acquise chez l'homme ou ceux de mammifères qu'ils sont susceptibles d'infecter, d'un muramylpeptide de formule : 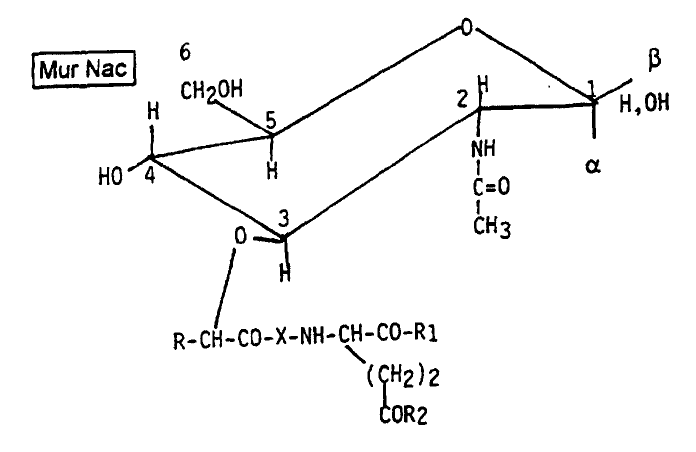 dans laquelle le groupe R est ou un groupe méthyle ; X est un résidu L-alanyle , et R1 est un groupe, amino ou un groupe O(CH2)ₓ H avec x = 1, 2, 3 ou 4, R2 est, indépendamment de R1, un groupe hydroxyle, amino, O(CH₂)ₓH avec x = 1, 2, 3 ou 4 et dans laquelle le muramylpeptide est apte à inhiber jusqu'à 100% la réplication de rétrovirus dans des cultures primaires, de monocytes de l'hôte.

Une sous-catégorie de muramylpeptides préférés pour la production des médicaments sus-indiqués est constituée par des muramylpeptides hydrophiles répondant à la formule générale sus-indiquée dans laquelle le groupe R est un hydrogène ou un groupe méthyle ; X est un résidu L-alanyle ou L-thréonyle, et R1 et R2 sont indépendamment l'un de l'autre, des groupes hydroxyle, amino, O(CH2)ₓ H avec x = 1, 2, 3 ou 4, étant entendu que, lorsque X est un résidu L-alanyle, l'un au moins de ces deux groupes R1 et R2 est toujours un groupe O(CH2)ₓ H tel que précédemment défini.

Des composés préférés pour l'utilisation selon l'invention sont le Murabutide (Nac-Mur-L-Ala-DGln OₙC₄H₉) et le Muramétide (Nac-Mur-L-Ala-DGln OMe). Ces molécules présentent un excellent profil d'activités chez l'homme ; elles sont dénuées d'effets secondaires et ont démontré leur très bonne tolérance, lors d'essais cliniques effectués chez des volontaires sains et des sujets cancéreux.

Il est à cet égard remarquable que les susdits muramylpeptides soient capables, à des concentrations relativement faibles, d'exercer une inhibition complète, jusqu'à 100 %, de la prolifération du VIH, dans des cultures primaires de monocytes, et ce plus particulièrement dans les protocoles expérimentaux auxquels il sera fait référence ci-après.

Il est particulièrement intéressant de remarquer que la manifestation de l'effet inhibiteur de ces muramylpeptides à l'égard de la réplication rétrovirale, n'est pas liée à une simultanéité d'infection des monocytes et de traitement de ces dernières avec ces muramylpeptides.

Des caractéristiques supplémentaires de l'invention apparaîtront encore au cours de la description qui suit, des effets biologiques exercés par deux muramylpeptides préférés à l'encontre de la réplication du VIH dans des cultures primaires de monocytes humains prélevés sur des volontaires sains.

Dans l'exemple 1, le Murabutide et le Muramétide ont démontré leur capacité d'inhiber la prolifération de macrophages en culture. Pour cela, des monocytes prélevés chez un donneur sont mis en culture pendant 5 jours soit a) sans stimulation (afin d'évaluer leur niveau de prolifération spontanée) soit b) en présence d'interleukine 3 recombinante humaine (rh IL-3) soit c) en présence et de rh IL-3 et de rh GM-CSF "granulocyte-macrophage colony stimulating factor" recombinant humain. Ces deux traitements permettent d'obtenir un haut niveau de prolifération. Les composés de l'invention sont ajoutés au milieu de culture un jour avant l'addition de thymidine tritiée (³H-thymidine). Les cellules en voie de division incorporent cette thymidine. Les cellules (qui se sont différenciées en macrophages pendant la durée de la culture) sont recueillies et lavées, et on évalue le niveau de prolifération en mesurant dans un compteur beta, la quantité de ³H incorporée suivant des méthodes classiques telles que décrites dans Blood, Vol. 76, pages 1490-1493, 1990. Les résultats sont présentés dans le tableau 1 et montrent que les deux dérivés sont capables, même à la dose de 1 µg/ml, d'inhiber la prolifération des macrophages stimulées par l'IL-₃ ou la combinaison IL-₃/GM-CSF. L'effet d'inhibition de la prolifération spontanée a été observé avec 10 µg/ml de Murabutide et 10 ou 50 µg/ml de Muramétide.

L'exemple 2 démontre l'effet du Murabutide et du Muramétide sur le niveau de réplication du VIH dans des cultures primaires de monocytes humains prélevés sur des volontaires sains. Des cultures monocytaires ont été infectées au jour 0 par une source VIH (HTLV III Ba-L) qui présente un tropisme pour les monocytes. Certaines cultures furent traitées par différentes concentrations des composés soit 1 jour avant, soit le même jour, soit 1 jour après l'inoculation par le VIH. La réplication du virus a été évaluée au jour 7 par la mesure de la quantité de protéine virale P24 dans les surnageants comme décrite dans Blood, Vol.76, page 1490-1493, 1990. Les résultats présentés dans le tableau 2 montrent clairement que le traitement par le Murabutide à une concentration de 10 à 50 µg/ml inhibe complètement la réplication virale que le traitement ait été pratiqué au jour -1, au jour 0 ou au jour +1 par rapport à l'infection. De façon similaire, le traitement par le Muramétide a permis d'observer une suppression hautement significative de la réplication virale et cet effet est de 100 % à la dose de 50 µg/ml quelque soit ici aussi le montant du traitement.

Ces résultats sont les premiers décrits ayant permis d'obtenir une inhibition complète par un muramylpeptide de la réplication de VIH dans des monocytes humains. Il faut souligner que l'inhibition s'obtient quand le composé est ajouté à la culture une seule fois et même après l'infection par VIH.

Les données précédentes montrent que les muramylpeptides de l'invention peuvent être appliquées à la constitution de médicaments applicables à la prévention ou le traitement du SIDA, ou des syndromes qui lui sont associés, par exemple le sarcome de Kaposi.

L'invention est également applicable à la constitution de médicaments dans lesquels les muramylpeptides sont utilisés en association avec d'autres agents thérapeutiques utilisés pour prévenir ou inhiber la prolifération et la diffusion du VIH chez l'homme. Parmi ces agents on peut citer les interférons α, β, γ et le GM-CSF.

Les molécules de l'invention peuvent être utilisées en clinique humaine soit à titre préventif chez des sujets à risques, soit à titre curatif chez des individus séropositifs avant l'apparition de signes cliniques ou des patients ayant développé des manifestations de SIDA. Les doses thérapeutiques du muramylpeptide (par exemple Murabutide ou Muramétide) à administrer soit seul, soit en association avec des traitements antiviraux, particulièrement des cytokines, se situent entre 1 µg et 500 µg/Kg/jour. L'administration peut être donnée par voie systémique, par injection sous-cutanée ou intraveineuse ou par perfusion. Le traitement peut consister en administrations journalières ou à quelques jours d'intervalle et se prolonger de une semaine à plusieurs mois suivant l'effet observé.

Dans le cas d'individus séropositifs ou malades, le traitement doit être prolongé jusqu'à absence de détection d'antigène ou de gènes viraux respectivement dans le sérum ou les cellules de l'individu infecté. Dans le cas d'individus à risque, le traitement préventif doit être appliqué pendant la période où il existe un risque d'infection.

Les molécules de l'invention ainsi que les autres molécules de la famille des muramylpeptides peuvent aussi être utilisées comme réactifs de laboratoire afin de permettre l'évaluation en tant qu'agents anti-VIH d'autres drogues présumées douées d'activité antivirale. Ainsi des doses suboptimales de muramylpeptides pourraient être utilisées en association avec un autre agent pour déceler une activité potentielle de ce dernier.

Ce type de réactif pourrait être utilisé dans des systèmes d'expérimentation in vitro mettant en oeuvre des cultures de monocytes/macrophages telles que décrites dans ce brevet ou des méthodes d'évaluation in vivo incluant l'utilisation de souris SCID.

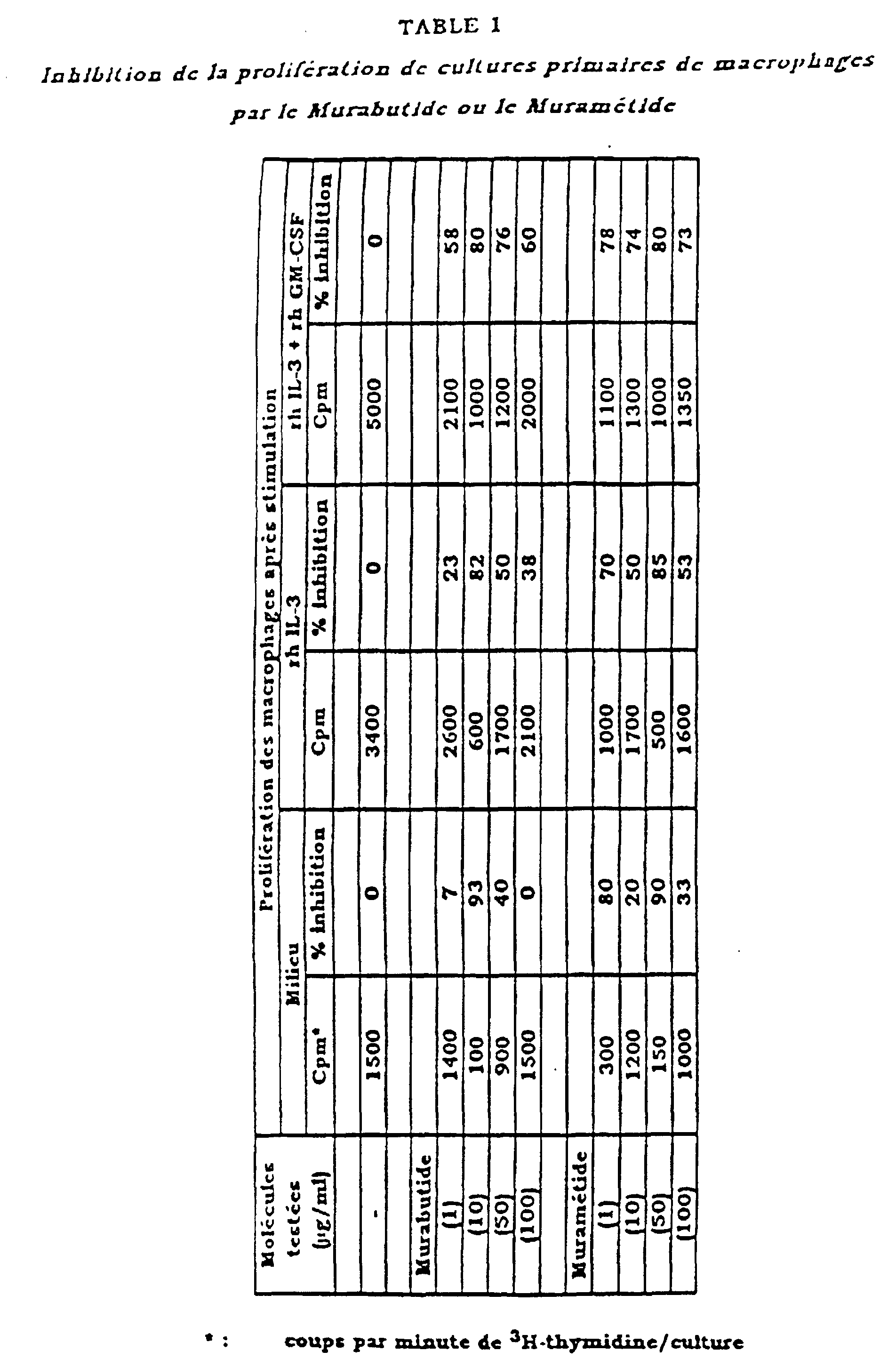

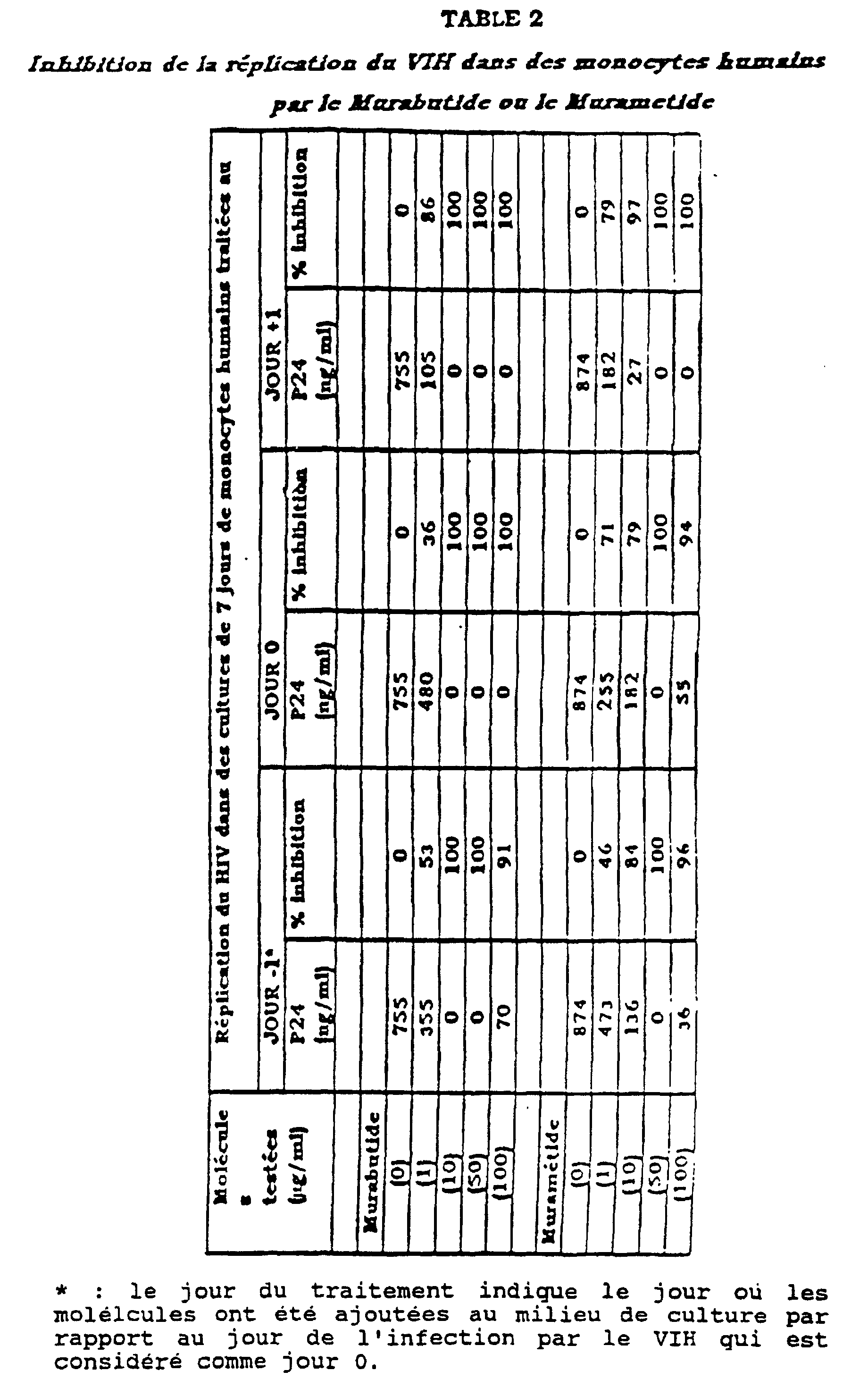

## Revendications

1. Utilisation pour la fabrication d'un médicament inhibant la réplication de rétrovirus de l'immunodéficience acquise chez l'homme ou les mammifères qu'ils sont susceptibles d'infecter, d'un muramylpeptide de formule: 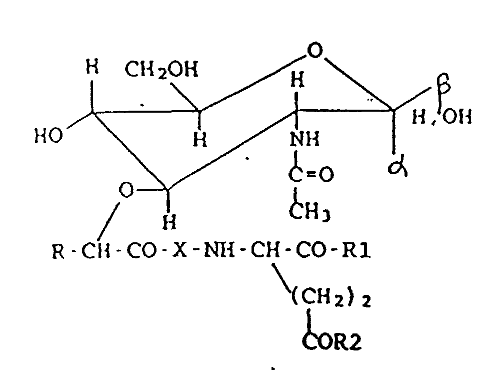 dans lequel le groupe R est un groupe méthyle; X est un résidu L-alanyle, et R1 est un groupe O(CH₂)ₓH avec x=1, 2, 3 ou 4, R2 est, indépendamment de R1 un groupe hydroxyl amino ou un groupe O (CH₂)ₓH avec x = 1, 2, 3 ou 4, et dans laquelle le muramylpeptide est apte à inhiber jusqu'à 100% la réplication de rétrovirus dans des cultures primaires de monocytes de l'hôte.

2. Utilisation selon la revendication 1, **caractérisée en ce que** le groupe R est un groupe méthyle, et le groupe R2 est un groupe NH₂.

3. Utilisation selon la revendication 1, **caractérisée en ce que** le muramylpeptide est le Muramétide.

4. Utilisation selon la revendication 1, **caractérisée en ce que** le muramylpeptide est le Murabutide.

5. Utilisation selon la revendication 1, **caractérisée en ce que** le muramyl peptide est le Muradimétide.

6. Utilisation selon l'une quelconque des revendications 1 à 5 pour la fabrication de médicaments destinés à la prévention ou au traitement du SIDA ou des syndromes qui lui sont associés, notamment le sarcome de Kaposi.

7. Utilisation selon la revendication 6, pour la fabrication de médicaments contenant en sus du susdit muramylpeptide, une autre molécule participant à l'action anti-rétrovirale.

8. Utilisation selon la revendication 7, **caractérisée en ce que** l'autre molécule est une cytokine, tel qu'un interféron α,β ou γ.

9. Utilisation selon la revendication 7, **caractérisée en ce que** l'autre molécule est le GM-CSF.

10. Utilisation selon la revendication 7, **caractérisée en ce que** l'autre molécule est un inhibiteur de protéase.

## Claims

1. Use, for the production of a drug inhibiting replication of acquired immunodeficiency retroviruses in human or mammals that they are likely to infect, of a muramyl peptide of the formula: 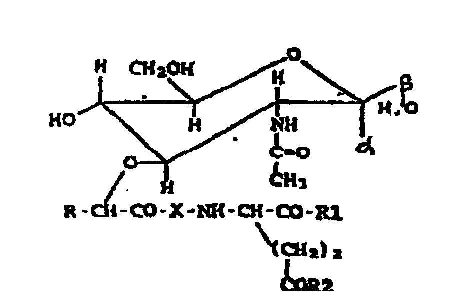 wherein the group R is a methyl group; X is an L-alanyl residue and R1 is a O (CH₂) ₓH group with x = 1, 2, 3 or 4, R2 independently of R1 is a hydroxyl, amino group or a O (CH₂)ₓH group with x = 1, 2, 3 or 4 and in which the muramyl peptide is suited to inhibit up to 100% the retrovirus replication in primary cultures of monocytes of the host.

2. Use according to Claim 1, **characterised in that** the group R is a methyl group and the group R2 is NH₂ group.

3. Use according to Claim 1, **characterised in that** the muramyl peptide is Murametide.

4. Use according to Claim 1, **characterised in that** the muramyl peptide is Murabutide.

5. Use according to Claim 1, **characterised in that** the muramyl peptide is Muradimetide.

6. Use according to any one of Claims 1 to 5 for the production of drugs intended to prevent or treat AIDS or syndromes associated therewith, notably the Kaposi sarcoma.

7. Use according to Claim 6 for the production of drugs containing in addition to the said muramyl peptide another molecule participating in the anti-retroviral action.

8. Use according to Claim 7, **characterised in that** the other molecule is a cytokine, such as an α, β or γ interferon.

9. Use according to claim 7, **characterised in that** the other molecule is GM-CSF.

10. Use according to Claim 7, **characterised in that** the other molecule is a protease inhibitor.

## Patentansprüche

1. Verwendung zur Herstellung eines Medikaments zur Inhibierung der Replikation von Retroviren der erworbenen Immunschwäche beim Menschen oder bei den Säugern, die angesteckt werden können, eines Muramylpeptids mit der Formel: 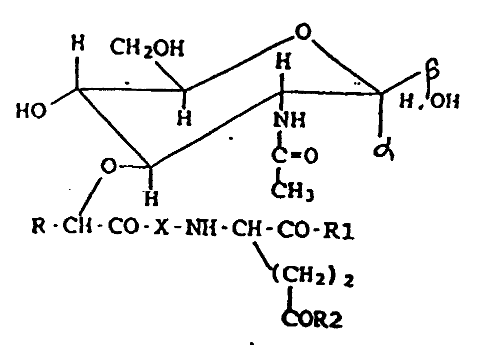 in welcher R eine Methylgruppe ist; X einen L-Alanylrest darstellt und R1 eine O(CH₂)ₓH Gruppe ist, wobei x = 1, 2, 3 oder 4 ist, R2 unabhängig von R1 eine Hydroxygruppe oder eine Aminogruppe oder eine O(CH₂)ₓH Gruppe ist, wobei x = 1, 2, 3 oder 4 ist und wobei das Muramylpeptid in der Lage ist, bis zu 100% der Replikation der Retroviren in primären Monocytenkulturen des Wirts zu inhibieren.

2. Verwendung nach Anspruch 1, **dadurch gekennzeichnet, dass** R eine Methylgruppe und R2 eine NH₂-Gruppe ist.

3. Verwendung nach Anspruch 1, **dadurch gekennzeichnet, dass** das Muramylpeptid Murametid ist.

4. Verwendung nach Anspruch 1, **dadurch gekennzeichnet, dass** das Muramylpeptid Murabutid ist.

5. Verwendung nach Anspruch 1, **dadurch gekennzeichnet, dass** das Muramylpeptid Muradimetid ist.

6. Verwendung nach einem der Ansprüche 1 bis 5 zur Herstellung von Medikamenten, die für die Prävention oder die Behandlung von AIDS oder den damit verbundenen Syndromen, insbesondere das Kaposi-Sarcom, bestimmt sind.

7. Verwendung nach Anspruch 6, zur Herstellung von Medikamenten, die außer dem obengenannten Muramylpeptid ein anderes Molekül enhalten, das an der anti-retroviralen Wirkung teilhat.

8. Verwendung nach Anspruch 7, **dadurch gekennzeichnet, dass** das andere Molekül ein Cytokin, wie z.B. ein α-, β- oder γ-Interferon, ist.

9. Verwendung nach Anspruch 7, **dadurch gekennzeichnet, dass** das andere Molekül das GM-CSF ist.

10. Verwendung nach Anspruch 7, **dadurch gekennzeichnet, dass** das andere Molekül ein Proteaseinhibitor ist.
